# EUROPÄISCHE PATENTSCHRIFT

(11) **EP 0 302 227 B1**
(45) Veröffentlichungstag und Bekanntmachung des Hinweises auf die Patenterteilung: **13.01.1993**
(21) Anmeldenummer: 88110464.0
(22) Anmeldetag: 30.06.1988
(51) Int. Cl.: C07D 213/78, C07D 401/04, C07D 213/73, C07D 213/74, C07D 213/75

(54) **5-Halogen-6-amino-nikotin-säurehalogenide, ihre Herstellung und ihre Verwendung**
5-Halo-6-amino-nicotinic acid halides, their preparation and their use
Halogénures d'acide 5-halo-6-amino-nicotinique, leur préparation et leur utilisation

(30) Priorität: 11.07.1987 DE 3723069
(43) Veröffentlichungstag der Anmeldung: 08.02.1989
(73) Patentinhaber: BAYER AG, 51368 Leverkusen (DE)
(72) Erfinder: Lindel, Hans, Dr., D-5090 Leverkusen 1 (DE); Hallenbach, Werner, Dr., D-4018 Langenfeld (DE)

(56) Entgegenhaltungen:
- EP-A- 0 244 728
- DE-A- 2 419 535
- US-A- 4 129 734
- US-A- 4 596 883
- PATENT ABSTRACTS OF JAPAN, Band 10, Nr. 239, 19. August 1986, Seite (C-367)(2295); & JP - A - 61 72 753
- HOUBEN-WEYL METHODEN DER ORGANISCHEN CHEMIE, 4. Auflage, Band VII/2b, Ketone Teil II, 1976, Georg Thieme Verlag, Stuttgart, DE
- HOUBEN-WEYL METHODEN DER ORGANISCHEN CHEMIE, 4. Auflage, Band VII/2a, Ketone Teil I, 1973, Georg Thieme Verlag, Stuttgart, DE

## Beschreibung

Die vorliegende Erfindung betrifft neue 5-Halogen-6-amino-nikotinsäurehalogenide, Verfahren zu ihrer Herstellung und ihre Verwendung zur Herstellung der entsprechenden 3-Pyridylalkylketone.

3-Pyridylalkylketone sind wertvolle Zwischenprodukte zur Herstellung leistungsfördernder Pyridylethanolamine. Die Ketone und ihre Herstellung sind in einer nicht vorveröffentlichten Anmeldung der Anmelderin (Deutsche Patentanmeldung P 36 15 293.5) allgemein beschrieben. Sie werden nach den dort beschriebenen Verfahren erhalten, indem man Nikotinsäurealkylester mit Essigsäurealkylester in einer Claisen'schen Esterkondensation umsetzt und den so erhaltenen Pyridoylessigester verseift und decarboxyliert.

Es wurde gefunden:
1. 5-Halogen-6-amino-nikotinsäurehalogenide der Formel (1) 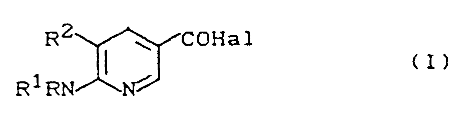 in welcher
   R für Wasserstoff oder C₁ -₃-Alkyl steht,
   R¹ für Wasserstoff, C₁ -₃-Alkyl der C₁ -₃-Alkanoyl steht,
   R und R¹ gemeinsam mit dem Stickstoffatom, an das sie gebunden sind. für einen 5- oder 6- gliedrigen heterocyclischen Rest stehen,
   _{R}² für Halogen steht,
   Hal für Halogen steht.
2. Verfahren zur Herstellung der 5-Halogen-6-amino-nikotinsäurehalogenide der Formel (I) 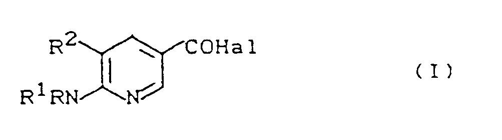 in welcher
   R, R¹, R², Hal die oben angegebene Bedeutung haben,
      dadurch gekennzeichnet, daß man 5-Halogen-6-amino-nikotinsäuren der Formel (II) 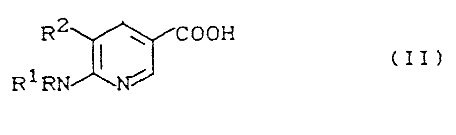 in welcher
   R, R¹, R² die oben angegebene Bedeutung haben,

   halogeniert.
3. Verwendung von 5-Halogen-6-amino-nikotinsäurehalogeniden der Formel (I) 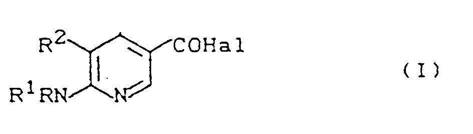 in welcher
   R, R¹, R², Hal die oben angegebene Bedeutung haben,
      zur Herstellung von 5-Acetylpyridinen der Formel (III) 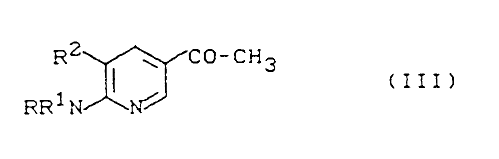 in welcher
   R, R¹ und R² die oben angegebene Bedeutung haben,

   dadurch gekennzeichnet, daS man 5-Halogen-6-amino-nikotinsäurehalogenide der Formel (I) mit einer Verbindung der Formel (IV) 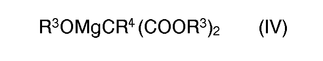 in welcher
   R³ für C₁₋₃-Alkyl steht
   _{R}⁴ für Wasserstoff steht,

   umsetzt und anschließend verseift und decarboxyliert.

Mit Hilfe der erfindungsgemäßen 5-Halogen-6-amino-nikotinsäurehalogenide sind die 3-Pyridylalkylketone und über diese die Pyridylethanolamine in guten Ausbeuten und ausgehend von preiswerten Ausgangsprodukten zugänglich.

Bevorzugt sind Verbindungen der Formel (I), in welcher
_{R}² für Chlor oder Brom steht,
R für Wasserstoff oder C₁-C₃-Alkyl steht,
R¹ für Wasserstoff, C₁-C₃-Alkyl oder C₁-C₃-Alkanoyl steht,

oder R und R¹ gemeinsam mit dem Stickstoffatom, an das sie gebunden sind, für einen heterocyclischen Rest stehen,
Hal für Chlor oder Brom steht.

Besonders bevorzugt sind Verbindungen der Formel (I), in der
R² für Chlor oder Brom steht,
Hal für Chlor steht,
R für Wasserstoff oder Methyl steht,
R¹ für Wasserstoff, Methyl oder Acetyl steht

oder R und R¹ gemeinsam mit dem Stickstoffatom, an das sie gebunden sind, für einen 5- oder 6-gliedrigen Ring wie Pyrrolidinyl, Pyrryl, Piperidinyl oder Morpholinyl stehen.

Im einzelnen seien folgende Verbindungen der Formel (I) genannt:
5-Brom-6-aminonikotinsäurechlorid,
5-Chlor-6-aminonikotinsäurechlorid,
5-Brom-6-methylaminonikotinsäurechlorid,
5-Chlor-6-acetaminonikotinsäurechlorid,
5-Brom-6-N-pyrrolidino-nikotinsäurechlorid,
5-Chlor-6-N-morpholino-nikotinsäurechlorid.

Setzt man bei Verfahren 2) als 5-Halogen-6-amino-nikotinsäure der Formel (11) 5-Brom-6-methylaminoni- kotinsäure und als Halogenierungsmittel Thionylchlorid ein, läßt sich die Reaktion durch das folgende Schema darstellen:

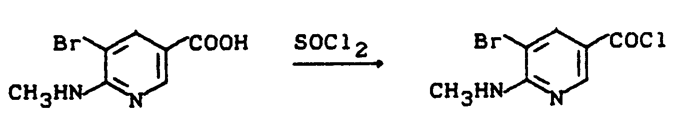

Die Verbindungen der Formel (11) sind zum Teil bekannt oder lassen sich analog zu bekannten Verfahren herstellen (Graf, J. prakt. Chem. (2) 138 (1933) S. 244). Bevorzugt sind Verbindungen der Formel (11), in welcher R, R¹ und R² die bei den Verbindungen der Formel (I) angegebenen Bedeutungen besitzen.

Im einzelnen seien die folgenden Verbindungen der Formel (11) genannt:
5-Brom-6-aminonikotinsäure,
5-Chlor-6-aminonikotinsäure,
5-Brom-6-methylaminonikotinsäure,
5-Chlor-6-acetaminonikotinsäure,
5-Brom-6-N-pyrrolidino-nikotinsäure,
5-Chlor-6-N-morpholino-nikotinsäure.

Als Halogenierungsmittel werden anorganische Säurechloride verwendet. Beispielhaft seien genannt: Phosphoroxychlorid, Phosphorpentachlorid, Thionylchlorid.

Die Reaktion wird durchgeführt, indem man eine Verbindung der Formel (II) mit 1 bis 1,5 Äquivalenten des anorganischen Säurechlorids, gegebenenfalls in einem Verdünnungsmittel, behandelt.

Die Umsetzung wird bei Temperaturen von 20 °C bis 150°C durchgeführt, es wird bevorzugt bei Normaldruck gearbeitet.

Als Verdünnungsmittel können alle inerten organischen Lösungsmittel verwendet werden. Hierzu zählen insbesondere aliphatische und aromatische gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran, Dioxan sowie Phosphoroxychlorid.

Setzt man bei Verfahren 3) als Verbindung der Formel (I) 5-Brom-6-N-pyrrolonikotinsäurechlorid und als Verbindung der Formel (IV) Methoxymagnesiummalonsäuredimethylester ein, läßt sich Verfahren 3) durch das folgende Reaktionsschema darstellen:

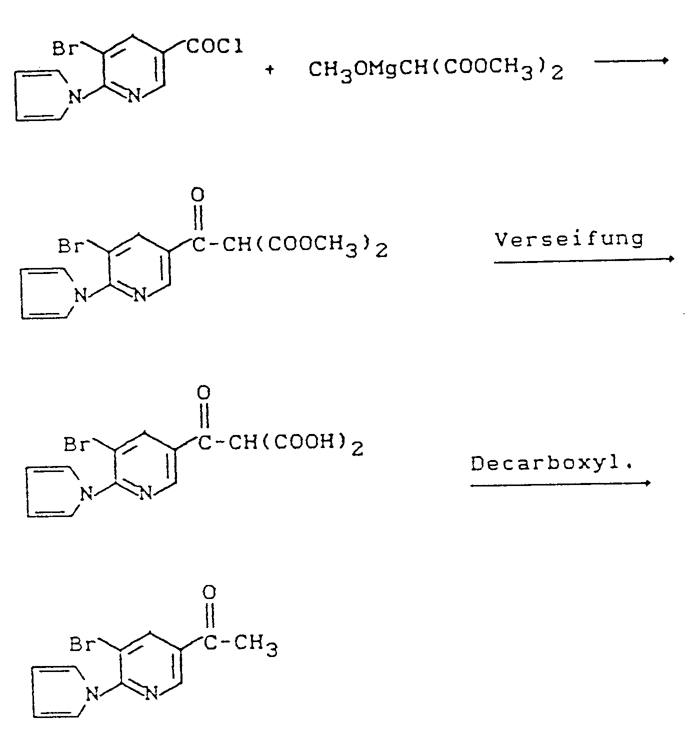

Verbindungen der Formel (IV) sind bekannt (Org. Synth. Coll. Vol. IV (1963), 285; Ber. dt. Chem. Ges. 67 (1934), 935).

Im einzelnen seien folgende Verbindungen der Formel (IV) genannt:
Methoxymagnesiummalonsäuredimethylester,
Ethoxymagnesiummalonsäurediethylester.

Verfahren 3) wird durchgeführt, indem man äquimolare Mengen der Verbindungen der Formeln (I) und (IV) in einem Verdünnungsmittel zur Reaktion bringt und anschließend verseift, wobei die β-Ketodicarbonsäure decarboxyliert. Als Verdünnungsmittel dienen alle inerten organischen Lösungsmittel. Hierzu zählen gegebenenfalls halogenierte aliphatische und aromatische Kohlenwasserstoffe wie Pentan, Hexan, Benzol, Toluol, Dichlormethan, Chloroform, Chlorbenzol; Ether wie Diethylether, Tetrahydrofuran; Alkohole wie Methanol, Ethanol. Die Reaktion wird bei Temperaturen von 20 ° C bis 150°C, bevorzugt bei der Siedetemperatur des verwendeten Lösungsmittels, durchgeführt.

Die Verseifung wird mit anorganischen Säuren wie Salzsäure oder Schwefelsäure, organischen Carbonsäuren wie Essigsäure oder Propionsäure durchgeführt. Es können auch Mischungen von anorganischen und organischen Säuren verwendet werden. Die Verseifung kann auch mit Basen wie alkoholischen oder wäßrigen Lösungen von Alkali- und Erdalkalihydroxiden oder -carbonaten z.B. Natrium-, Kalium-, Bariumhydroxid, Natrium-, Kaliumcarbonat durchgeführt werden.

Die nach dem erfindungsgemäßen Verfahren 3) erhältlichen 2-Amino-3-halogen-5-acetylpyridine werden zur Herstellung von Pyridinethanolaminderivaten verwendet. Dazu werden die Acetylpyridine mit elementarem Halogen oder mit Kupferhalogeniden umgesetzt. Die dabei erhältlichen Halogenmethylpyridylketone werden anschließend mit Amimen umgesetzt, und die dabei erhaltenen Pyridyl-aminomethylketone reduziert. Diese Reaktion läßt sich durch folgendes Formelschema illustrieren:

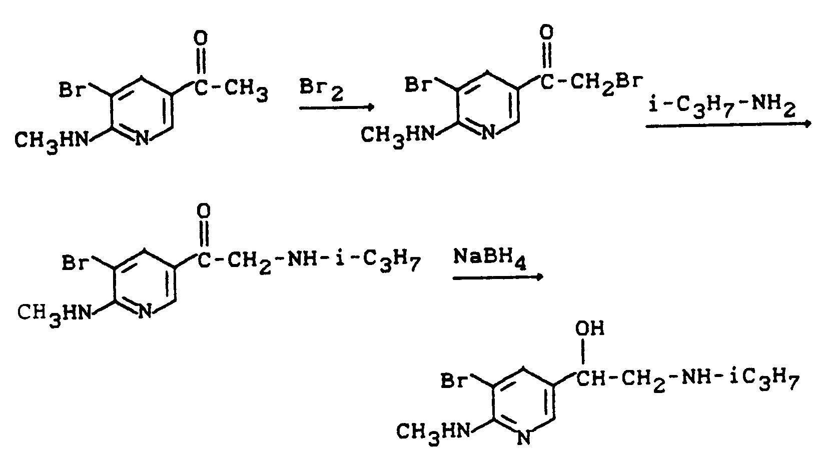

Die Halogenmethylpyridylketone können auch zuerst zu den Pyridylhalogenethanolen reduziert und anschließend mit Aminen zu Pyridylethanolaminen umgesetzt werden.

Die Durchführung dieser Reaktionen ist in der oben erwähnten nicht vorveröffentlichten Anmeldung der Anmelderin Deutsche Patentanmeldung P 36 15 293.5 beschrieben.

### Herstellungsbeispiele

### Beispiel für Verfahren 2)

### 5-Chlor-6-amino-nikotinsäurechlorid

6,3 g (36 mmol) 5-Chlor-6-amino-nikotinsäure werden in 60 ml Thionylchlorid 4 Stunden unter Rückfluß erhitzt. Nach Abdestillieren der flüchtigen Bestandteile wird der Rückstand mit 50 ml Toluol versetzt und eingedampft. Man erhalt 6,9 g 5-Chlor-6-amino-nikotinsäurechlorid als Kristalle vom Fp.: >250 °C.

### Beispiel für Verfahren 3)

### 2-Amino-3-chlor-5-acetylpyridin

Zur siedenden Lösung von 8,55 g (37,5 mmol) Ethoxymagnesiummalonsäurediethylester (hergestellt nach Org. Synth. Coll. Vo. IV (1963), 285) in 120 ml abs. Tetrahydrofuran werden 7,2 g (37,5 mmol) 5-Chlor-6-aminonikotinsäurechlorid in 10 ml abs. Tetrahydrofuran gegeben, die Mischung wird 2 Stunden unter Rückfluß erhitzt. Nach Neutralisation mit 2N Schwefelsäure wird die organische Phase abgetrennt und eingedampft. Der Rückstand wird in einer Mischung aus 30 ml Eisessig, 20 ml wasser und 5 ml konz. Schwefelsäure 4 Stunden unter Rückfluß erhitzt. Der Ansatz wird in Eiswasser eingerührt, auf pH 4 gestellt und mit Essigester extrahiert. Nach Trocknen und Eindampfen erhält man 5,4 g (85 %) der Titelverbindung, Fp.: 188 ° C.

### Beispiel für die Herstellung der Halogenmethylpyridylketone

### Beispiel a

### 2-Amino-3-chlor-5-pyridyl-(brommethyl)keton

Zu einer Lösung von 17,0 g (0,1 mol) 2-Amino-3-chlor-5-acetylpyridin in einer Mischung von 19,3 g Bromwasserstoff (47 %ige wäßrige Lösung; 0,11 mol) und 500 ml Eisessig werden 16 g (0,1 mol) Brom getropft. Man rührt zwei Stunden nach, stellt die Mischung auf pH 8 und extrahiert mit Essigester. Nach Trocknen und Eindampfen erhält man 18,5 g (74 %) der Titelverbindung, Fp. 134°C.

### Beispiel für die Herstellung der Pyridyl-aminomethylketone

### Beispiel b

### 2-Amino-3-chlor-5-pyridyl-(isopropylaminomethyl)keton

In eine Lösung von 11,8 g (0,2 mol) Isopropylamin in 150 ml Methanol werden bei 0°C portionsweise 9,98 g (0,04 mol) der unter Beispiel a hergestellten Verbindung eingetragen. Man läßt auf Raumtemperatur kommen, rührt zwei Stunden nach und dampft ein. Der Rückstand wird in Puffer pH 5 aufgenommen und mit Ether gewaschen. Die wäßrige Phase wird auf pH 9 gestellt und mit Essigester extrahiert. Nach Trocknen und Eindampfen werden 6,8 g (75 %) der Titelverbindung als amorphes Pulver erhalten.

### Beispiel für die Herstellung der Pyridylethanolamine

### Beispiel c

### 1-(2-Amino-3-chlor-5-pyridyl)-2-isopropylaminoethanol

Zur Lösung von 2,28 g (10 mmol) der nach Beispiel b hergestellten Verbindung in 50 ml Methanol werden bei 0 °C portionsweise 0,38 g (10 mmol) Natriumborhydrid gegeben. Man läßt auf Raumtemperatur kommen, stellt mit verdünnter Salzsäure auf pH 1 und dampft ein. Der Rückstand wird in Wasser aufgenommen und mit Ether gewaschen. Dann wird auf pH 10 gestellt und mit Essigester extrahiert. Nach Trocknen und Eindampfen erhält man 2,1 g (92 %) der Titelverbindung, Fp. 146 ° C.

## Patentansprüche (Patentansprüche für folgende(n) Vertragsstaat(en) : AT, BE, CH, DE, FR, GB, IT, LI, NL)

1. 5-Halogen-6-amino-nikotinsäurehalogenide der Formel (I) 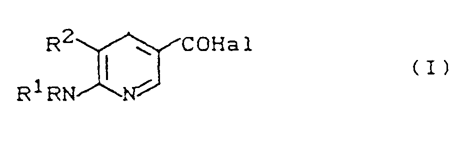 in welcher
R für Wasserstoff oder C₁ -₃-Alkyl steht,
R¹ für Wasserstoff, C₁ -₃-Alkyl oder C₁ -₃-Alkanoyl steht,
R und R¹ gemeinsam mit dem Stickstoffatom, an das sie gebunden sind, für einen 5- oder 6- gliedrigen heterocyclischen Rest stehen,
_{R}² für Halogen steht,
Hal für Halogen steht.

2. Verfahren zur Herstellung der 5-Halogen-6-amino-nikotinsäurehalogenide der Formel (I) 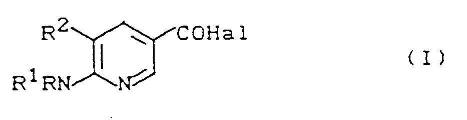 in welcher
R, R¹, R², Hal die in Anspruch 1 angegebene Bedeutung haben,
dadurch gekennzeichnet, daß man 5-Halogen-6-amino-nikotinsäuren der Formel (II) 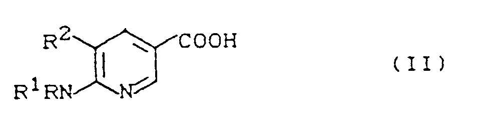 in welcher
R, R¹, R² die oben angegebene Bedeutung haben,
halogeniert.

3. Verwendung von 5-Halogen-6-amino-nikotinsäurehalogeniden der Formel (I) 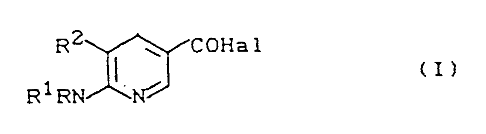 in welcher
R, R¹, R², Hal die in Anspruch 1 angegebene Bedeutung haben,
zur Herstellung von 5-Acetylpyridinen der Formel (III) 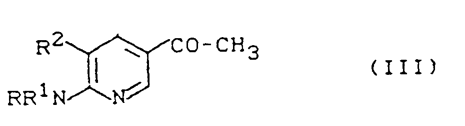 in welcher
R, R¹ und R² die oben angegebene Bedeutung haben,
dadurch gekennzeichnet, daß man 5-Halogen-6-amino-nikotinsäurehalogenide der Formel (I) mit einer Verbindung der Formel (IV) 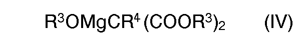 in welcher
R³ für C₁₋₃-Alkyl steht,
_{R}⁴ für Wasserstoff steht,
umsetzt und anschließend verseift und decarboxyliert.

## Patentansprüche (Patentansprüche für folgende(n) Vertragsstaat(en) : ES)

1. Verfahren zur Herstellung der 5-Halogen-6-amino-nikotinsäurehalogenide der Formel (I) 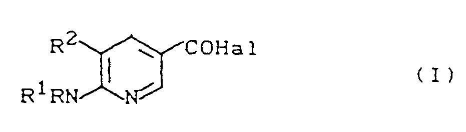 in welcher
R für Wasserstoff oder C₁ -₃-Alkyl steht,
R¹ für Wasserstoff, C₁ -₃-Alkyl oder C₁ -₃-Alkanoyl,
R und R¹ gemeinsam mit dem Stickstoffatom, an das sie gebunden sind, für einen 5- oder 6- gliedrigen heterocyclischen Rest stehen,
_{R}2 für Halogen steht,
Hal für Halogen steht,
dadurch gekennzeichnet, daß man 5-Halogen-6-amino-nikotinsäuren der Formel (II) 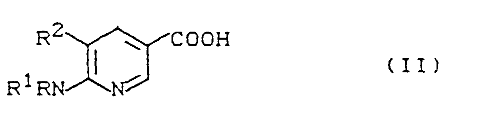 in welcher
R, R¹, R² die oben angegebene Bedeutung haben,
halogeniert.

2. Verwendung von 5-Halogen-6-amino-nikotinsäurehalogeniden der Formel (I) 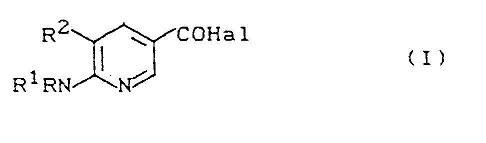 in welcher
R, R¹, R², Hal die in Anspruch 1 angegebene Bedeutung haben,
zur Herstellung von 5-Acetylpyridinen der Formel (III) 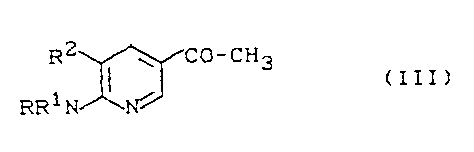 in welcher
R, R¹ und R² die oben angegebene Bedeutung haben,
dadurch gekennzeichnet, daß man 5-Halogen-6-amino-nikotinsäurehalogenide der Formel (I) mit einer Verbindung der Formel (IV) 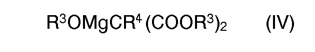 in welcher
R³ für C₁₋₃-Alkyl steht,
_{R}⁴ für Wasserstoff steht,
umsetzt und anschließend verseift und decarboxyliert.

## Claims (Claims for the following Contracting State(s) : AT, BE, CH, DE, FR, GB, IT, LI, NL)

1. 5-Halogeno-6-amino-nicotinic acid halides of the formula (I) 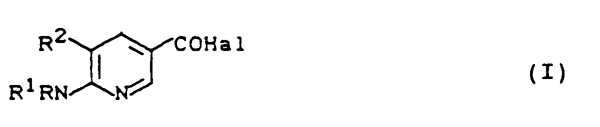 in which
R represents hydrogen or C₁₋₃-alkyl,
R¹ represents hydrogen, C₁₋₃-alkyl or C₁₋₃-alkanoyl or
Rand R¹, together with the nitrogen atom to which they are bonded, represent a 5- or 6- membered heterocyclic radical,
R² represents halogen and
Hal represents halogen.

2. Process for the preparation of the 5-halogeno-6-amino-nicotinic acid halides of the formula (I) 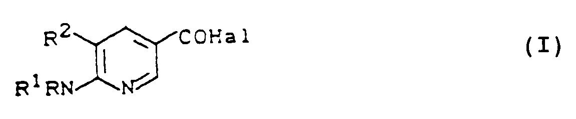 in which
R, R¹, R² and Hal have the meaning mentioned in Claim 1,
characterized in that 5-halogeno-6-amino-nicotinic acids of the formula (II) 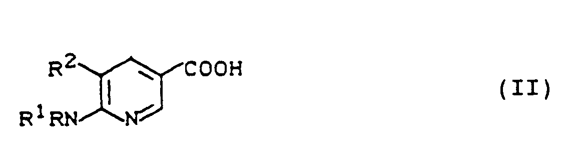 in which
R, R¹ and R² have the abovementioned meaning, are halogenated.

3. Use of 5-halogeno-6-amino-nicotinic acid halides of the formula (I) 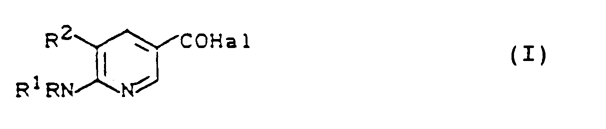 in which
R, R¹, R² and Hal have the meaning mentioned in Claim 1,
for the preparation of 5-acetylpyridines of the formula (III) 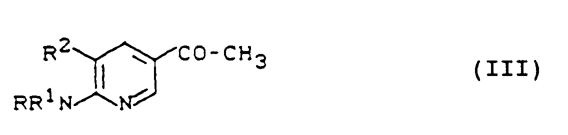 in which
R, R¹ and R² have the abovementioned meaning, characterized in that 5-halogeno-6-amino-nicotinic acid halides of the formula (I) are reacted with a compound of the formula (IV) 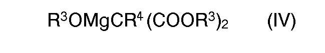 in which
R³ represents C₁₋₃-alkyl and
R⁴ represents hydrogen,
and the products are then hydrolysed and decarboxylated.

## Claims (Claims for the following Contracting State(s) : ES)

1. Process for the preparation of the 5-halogeno-6-amino-nicotinic acid halides of the formula (I) 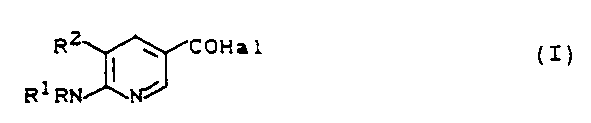 in which
R represents hydrogen or C₁₋₃-alkyl,
R¹ represents hydrogen, C₁₋₃-alkyl or C₁₋₃-alkanoyl or
Rand R¹, together with the nitrogen atom to which they are bonded, represent a 5- or 6- membered heterocyclic radical,
R² represents halogen and
Hal represents halogen,
characterized in that 5-halogeno-6-amino-nicotinic acids of the formula (II) 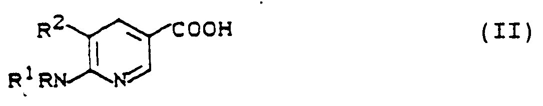 in which
R, R¹ and R² have the abovementioned meaning, are halogenated.

2. Use of 5-halogeno-6-amino-nicotinic acid halides of the formula (I) 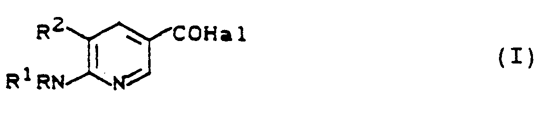 in which
R, R¹, R² and Hal have the meanings mentioned in Claim 1,
for the preparation of 5-acetylpyridines of the formula (III) 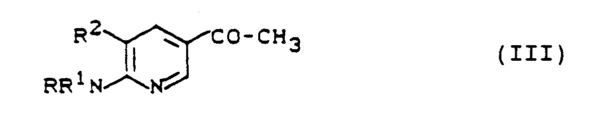 in which
R, R¹ and R² have the abovementioned meaning, characterized in that 5-halogeno-6-amino-nicotinic acid halides of the formula (I) are reacted with a compound of the formula (IV) 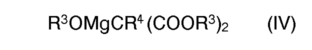 in which
R³ represents C₁₋₃-alkyl and
R⁴ represents hydrogen,
and the products are then hydrolysed and decarboxylated.

## Revendications (Revendications pour l'(les) Etat(s) contractant(s) suivant(s) : suivants : AT, BE, CH, DE, FR, GB, IT, LI, NL)

1. Halogénures d'acides 5-halogéno-6-amino-nicotiniques de formule I 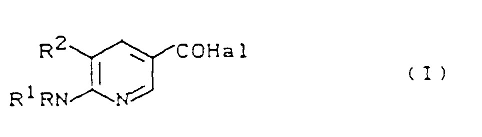 dans laquelle
R représente l'hydrogène ou un groupe alkyle en C₁-C₃,
R¹ représente l'hydrogène, un groupe alkyle en C₁-C₃ ou alcanoyle en C₁-C₃,
R et R¹ peuvent également former ensemble et avec l'atome d'azote auquel ils sont reliés un groupe hétérocyclique à 5 ou 6 chaînons,
R² représente un halogène,
Hal représente un halogène.

2. Procédé de préparation des halogénures d'acides 5-halogéno-6-amino-nicotiniques de formule I 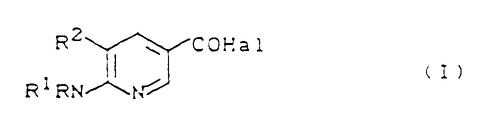 dans laquelle R, R¹, R² Hal ont les significations indiquées dans la revendication 1, caractérisé en ce que l'on soumet des acides 5-halogéno-6-amino-nicotiniques de formule II 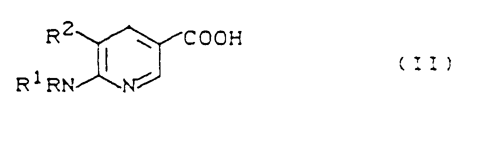 dans laquelle R, R¹, R² ont les significations indiquées ci-dessus, à halogénation.

3. Utilisation des halogénures d'acides 5-halogéno-6-amino-nicotiniques de formule I 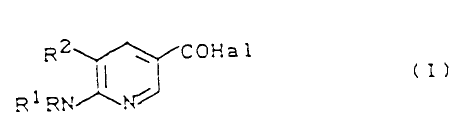 dans laquelle R, R¹, R², Hal ont les significations indiquées ci-dessus, pour la préparation des 5-acétylpyridines de formule III 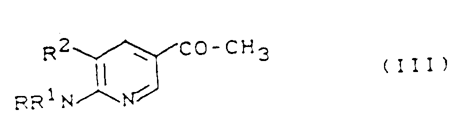 dans laquelle R, R¹, R² ont les significations indiquées ci-dessus, caractérisée en ce que l'on fait réagir les halogénures d'acides 5-halogéno-6-amino-nicotiniques de formule I avec un composé de formule IV 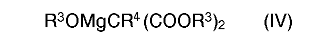 dans laquelle
R³ représente un groupe alkyle en C₁-C₃,
R⁴ représente l'hydrogène,
puis on soumet à saponification et décarboxylation.

## Revendications (Revendications pour l'(les) Etat(s) contractant(s) suivant(s) : suivant : ES)

1. Procédé de préparation des halogénures d'acides 5-halogéno-6-amino-nicotiniques de formule I 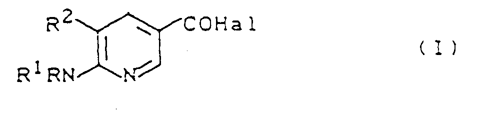 dans laquelle
R représente l'hydrogène ou un groupe alkyle en C₁₋₃,
R¹ représente l'hydrogène, un groupe alkyle en C₁-C₃ ou alcanoyle en C₁-C₃,
R et R¹ peuvent également former ensemble et avec l'atome d'azote auquel ils sont reliés un groupe hétérocyclique à 5 ou 6 chaînons,
R² représente un halogène,
Hal représente un halogène,
caractérisé en ce que l'on soumet des acides 5-halogéno-6-amino-nicotiniques de formule II 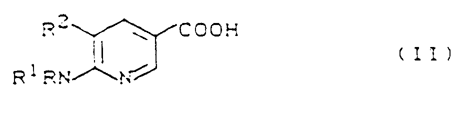 dans laquelle R, R¹, R² ont les significations indiquées ci-dessus, à halogénation.

2. Utilisation des halogénures d'acides 5-halogéno-6-amino-nicotiniques de formule I 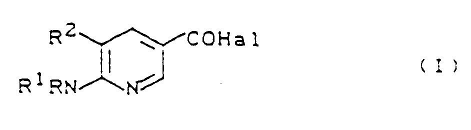 dans laquelle R, R¹, R², Hal ont les significations indiquées dans la revendication 1 pour la préparation des 5-acétylpyridines de formule III 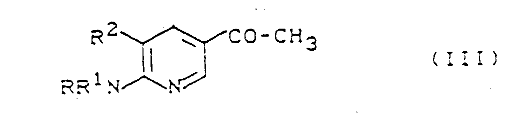 dans laquelle R, R¹ et R² ont les significations indiquées ci-dessus,
caractérisée en ce que l'on fait réagir les halogénures d'acides 5-halogéno-6-amino-nicotiniques de formule I avec un composé de formule IV 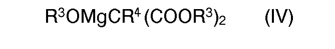 dans laquelle
R³ représente un groupe alkyle en Ci -C₃
R⁴ représente l'hydrogène,
puis on soumet à saponification et décarboxylation.
